# EUROPEAN PATENT SPECIFICATION

(11) **EP 2 217 291 B1**
(45) Date of publication and mention of the grant of the patent: **18.04.2012**
(21) Application number: 08849556.9
(22) Date of filing: 13.11.2008
(51) Int. Cl.: A61L 27/04, A61L 27/30

(54) **ONE PIECE ENDOSSEOUS TITANIUM OR ZIRCONIA DENTAL IMPLANT BEARING A BONDED CERAMIC OR GLASS-CERAMIC TRANSMUCOSAL ABUTMENT COMPONENT**
EINTEILIGES ENDOSSEALES TITAN- ODER ZIRKONIUMDIOXID-ZAHNIMPLANTAT MIT EINER TRANSMUKOSALEN VERBUNDENEN KERAMIK- ODER GLASKERAMIK-WIDERLAGERKOMPONENTE
IMPLANT DENTAIRE ENDO-OSSEUX MONOBLOC À BASE DE TITANE OU DE ZIRCONE PORTANT UN COMPOSANT SOUDÉ DE POINT D'APPUI TRANSMUCOSAL À BASE DE CÉRAMIQUE OU DE VITROCÉRAME

(30) Priority: 16.11.2007 GR 20070100691
(43) Date of publication of application: 18.08.2010
(73) Proprietor: Tripodakis, Aris-Petros, 115 28 Athens (GR); Iliadis, Georgios, 115 27 Athens (GR)
(72) Inventor: Tripodakis, Aris-Petros, 115 28 Athens (GR); Iliadis, Georgios, 115 27 Athens (GR)
(74) Representative: Karakatsanis, Georgios
(86) International application number: PCT/GR2008/000066
(87) International publication number: WO 2009/063259

(56) References cited:
- EP-A- 1 825 828
- WO-A-01/34056
- WO-A-2007/025784
- FR-A- 2 350 090

## Description

Technical field: A one-piece endosseous/transmucosal dental implant, bearing a thick tooth colored coating bonded on the abutment extention, to be used for the esthetic prosthetic restoration of missing teeth by means of a one stage surgical procedure.

**Background of the invention:** Dental implants have been successfully used for the replacement of missing teeth. Usually they have been applied by surgical procedure in two faces:
- The initial surgical procedure involves the endosseous placement of the implants underneath the mucosa of the edentulous area.
- The second stage surgery involves their mucosal uncovering and connection with the abutment on which the dental prosthesis is fixed.

The different types of dental implants that have been introduced thus far, mainly consisting of Ti, can be mechanically connected in the second stage with a separate transmucosal abutment component also consisting of Ti, supporting the fixation of the artificial teeth. Such a connection inherently and inevitably has the mechanical drawback of the eventual loosening or breakage of the retaining screw. Furthermore such a connection has also the biological drawback of the implant-abutment junction's micro gap, which acts as a bacterial trap.

The esthetic demands of the cervical part of the restorations call for biocompatible transmucosal materials that have the appearance and the optical behaviour comparable to the natural tooth substances. Thus an assortment of ceramic abutments has been introduced. These abutments, with varying mechanical properties, adopted the design originally applied in Ti abutments. They consist of a hollow tube basic form that provides access for the head of a connecting screw that stabilizes the abutment on the implant. This design while effective for the mechanical properties of a metallic abutment, unavoidably introduces severe tensile stresses in the area around the head of the retentive screw when lateral forces are applied on the abutment during function. Such stresses are incompatible and at times detrimental for the mechanical properties of ceramic materials that can successfully endure compressive stresses but are extremely vulnerable upon tensile stresses. To overcome this inadequacy the use transmucosal abutments made of stronger tooth colored materials such as pure Al₂O₃ or ZrO₂ was introduced. Nevertheless the extreme toughness of these materials prohibits intraoral shape modifications, while their strength is expected to be reduced after prolonged use. Moreover their optical quality involves unfavorable opacity.

The need for a revised design applied in conjunction with softer and easier to clinically modify ceramic materials combined with biologic, mechanical and esthetic improvements is therefore understandable. These requirements call for softer ceramic materials possessing optical qualities and depth of translucency similar to natural tooth substances, brought in the transmucosal and coronal area by a design that would eliminate tensile and favor compressive stresses in combination with the elimination of the implant- abutment connection drawbacks.

One stage surgery for implant placement (the endosseous implant placement being immediately followed by the transmucosal abutment connection, combined or not with the installation of an immediate provisional restoration) made possible the use of one piece implant/abutment fixtures. They basically involve an endosseous part possessing a rough threaded surface that results coronally to an implant head with a smooth metal collar large in height, designated for soft tissue contact. The implant head (cylindrical, conical, tapered or scalloped) is joined either directly to the prosthetic superstructure or to an other intermediate coronal part, metallic or ceramic. These designs fully uphold the mechanical drawback of the screw joint. Furthermore the biological drawback of the implant-abutment junction's micro gap, while still present, is positioned farther coronally, away from the bone level but still quite deep under the soft tissues.

Another category of one stage implants designed for immediate fixation of a provisional restoration and loading involves one piece implant/abutment fixtures that extend coronally beyond the transmucosal part, to a prosthetic head for the direct installation of the restoration. One international application of such design has already been published under the Patent Cooperation Treaty ("Immediate-load dental implants" by Kahdemann S. and Trenkler C., PCT: WO 20071025784 A2), and another one under the European Patent office ("One-part implant with hydroxylated soft tissue contact surface" by Schwarz F. et al., EP1 825 828A1).Moreover, one-piece dental implants in both Ti (i.e. NobelDirect, NobelBiocare, Gotteborg, Sweden) and Zirconia (i.e. whiteSKY Zirconium implant, bredent medical, Senden, Germany) are presently commercially available.

This latter approach eliminates the implant/abutment junction and fully abolishes all previously mentioned drawbacks. Nevertheless the abutment and coronal part are a direct extension of the implant material, Ti or Zirconia. In both occasions the endosseous material is one and the same with this extension. Therefore the metallic extension of a Ti implant does not meet the esthetic requirements of the soft tissues. Furthermore, if the implant material is Zirconia, its transmucosal element is too tough to modify intraorally, while its opacity is optically unfavorable. Moreover the position, inclination, size and height of its prefabricated crown retaining intraoral projection can effectively meet the clinical requirements of a restorable abutment only coincidentally, providing minimal leeway for the required necessary modifications by the existing clinical anatomical conditions. In addition, its predetermined flat finish line for the crown margin does not have the ability to follow the periimplant soft tissue crest in direct and intimate relationship.

These shortcomings could be overcome by a design that involves a one piece implant/abutment fixture that extends coronally beyond the transmucosal part, to a prosthetic head incorporating an amendable by intraoral grinding esthetic material for the direct installation of the restoration. Such an approach has appeared in an international application that has been published under the Patent Cooperation Treaty ("One-part dental implant" by Beekmans J.C.S., PCT: WO01/34056A1).That invention refers to a tooth shaped one-piece ceramic implant bearing a softer ceramic coating coronally. Its endosseous element tapers to a root form with oval cross-section that can only be fitted into the jaw bone only by punching. Therefore the mechanical anchorage of the endosseous part not being attained by screw retention is simply limited to friction and thus the acquired initial stability of the implant is expected to be inadequate for immediate loading. Furthermore, no reference has been made regarding the nature of the interface between the supporting core and the ceramic coating. Possibly, these are the reasons why the invention that dates since 2001, not being effective, has not been commercially introduced.

**The present invention** involves a one-piece Ti or Zirconia implant incorporating an endosseous screw-shaped part with a transmucosal abutment-coronal extension that bears a thick bonded ceramic or glass-ceramic coating. The moderate toughness of this coating ensures the ease of its intraoral modification to accommodate a superimposed restoration and provides the necessary optical qualities and biocompatibility in the cervical soft tissue environment. The coating is bonded either by an adhesive media or it is fired directly on the implant extension, fused or heat pressed. Thus the implant extension is acting as the supporting core of the abutment and crown bearing part and as the solid unifying element of the one-piece hybrid dental implant.

**The aim of the invention** is to combine the advantages of a one piece dental implant with the presence of a bonded transmucosal esthetic coating with mechanical properties that ensure the ease of its intraoral modification. The coating acquires the necessary mechanical endurance by assuming the appropriate design that allows the exertion of exclusively compressive forces on the fragile ceramic or glass-ceramic material.

**The advantages of the invention** are described as follows:

Biologic advantages: The fixture consists of one piece, while the interface among the implant material and the abutment coating is fully sealed due to the acquired bond, therefore the implant-abutment junction's micro gap acting as a bacterial trap does not exist. The retrieval or interchange of the transmucosal element is also not possible and the adverse biological consequence of the disruption of the intimate soft tissue adaptation is eliminated. Moreover the preparation finish-line that will accommodate the definitive crown margins is finalized by intraoral preparation of the coating after the healing and maturation of the soft tissue has been fully accomplished. Thus the crown margins can attain a biologically ideal, direct and intimate relationship with the periimplant soft tissue, after the architecture of its free gingival margin is completely stabilized.

Mechanical advantages: The absence of a screw joint between implant and abutment eliminates the common mechanical problems of screw loosening or fracture. The tooth colored ceramic or glass-ceramic coating material is bonded, fused or heat pressed on the strong Ti or Zirconia core extension of the implant. Therefore all the hazardous tensile forces are eliminated and the esthetic ceramic material is exposed exclusively to the tolerable compressive forces. Moreover the ceramic material that covers the core extension coronally due to its reduced toughness can be easily modified by intraoral preparation in order to meet the morphological clinical prerequisites of a restorable abutment for a full coverage restoration.

Esthetic advantages: The dark shade created on the soft tissues by the transmucosal metallic Ti surface is eliminated as it is covered by the ceramic coating material. The translucent ceramic material emerging through the transparent soft tissue crest allows its internal illumination by transmitting the light from the crown to the transmucosal part in a similar manner as the natural tooth illuminates its root and the adjacent gingival margin. Moreover the feasible intraoral preparation meets the esthetic prerequisites of a mildly sub-crestal finish line to accommodate the definitive crown margins in an esthetic relationship with the periimplant soft tissues.

**The description of the invention** is linked separately to its three different area levels:

In the endosseous part the implant can adopt most of the already commercially available and clinically successful configurations of regular dental implants designed for immediate loading. Namely it ought to be screw type, cylindrical or root formed, in order to attain adequate primary stability achieved upon insertion by self-taping into the jaw bone. Its surface ought to be rough in order to rapidly promote osseointegration. Its length can vary from 10 to 15 mm and its width from 3.25 to 5 mm. It may consist of a strong material either commercially pure Ti, or Ti alloy or Zirconia.

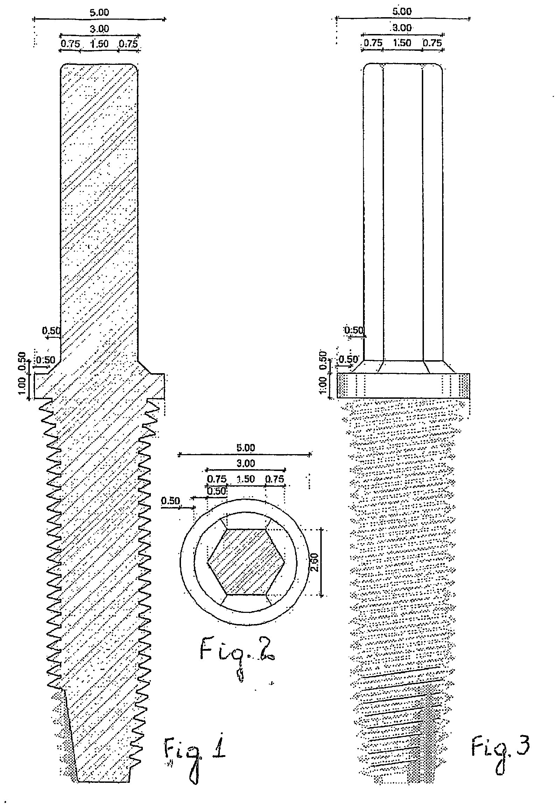

In the transmucosal part the fixture consists of a cervical part adjacent to the osseous crest and a soft tissue part. The endosseous material immediately above the bone crest widens forming a platform that aims to prevent over-screwing the implant into the prepared osseous bed during its insertion. For this purpose it has an increased diameter that varies from 3.5 to 4.5 mm. The platform contains a cylindrical or tapered collar of a varying height from 1.0 to 4.00 mm. The fixtures with the lower collar height in this area are to be used in the healed edentulous sites, whereas the ones with the higher collar height in the immediate extraction sites where the implant is seated in a deeper position into the socket. The surface of the collar is smooth and designated for the soft tissue contact (fig. 1 and 3).

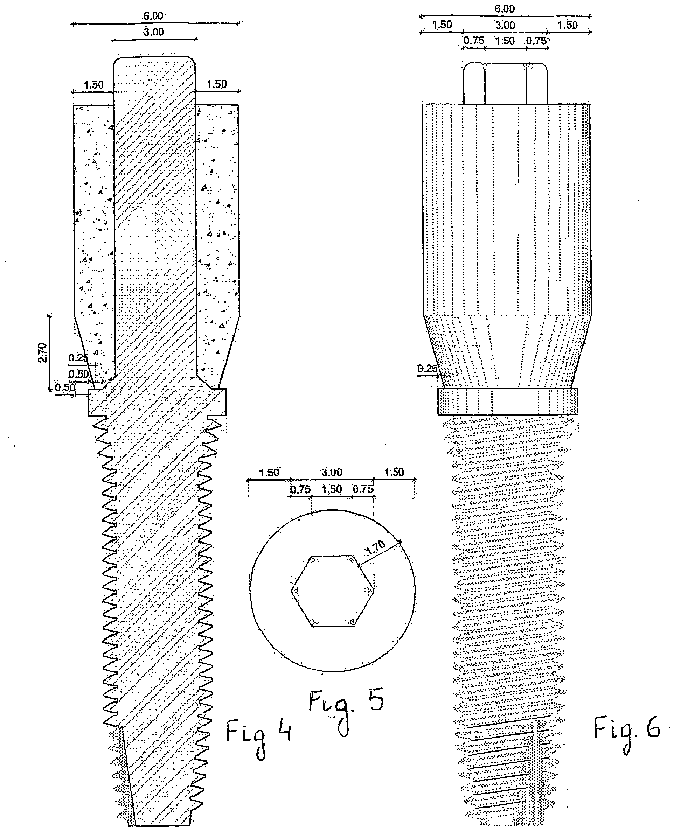

Immediately above the bone crest collar the material of the endosseous part of the fixture Ti or Zirconia is extended coronally forming a cylindrical or prismatic projection (i.e. hexagonal) of reduced diameter of 2.5 to 3.0 mm. The implant extension acts as the supporting core of the abutment and the solid unifying element between the endosseous and the restorative part of the hybrid implant. The connection of the extension with the main body of the implant slopes and widens forming a foundation base, in order to attain increased strength (fig. 1-3). Moreover the prismatic morphology of the extension acts ant-rotationally for the bonded coating. The extension is covered by a ceramic or glass-ceramic coating that is bonded, fused or heat pressed on the Ti or Zirconia core material. The thickness of the coating material becomes gradually increasing starting from 0.5 mm thus forming a platform switch cervically of 0.5 mm in order to prevent the potential crestal bone resorption around the material interface (diameter difference of the platform of the endosseous head and the emerging cervical veneered part of the transmucosal abutment). The coating material reaches its maximum thickness of 1.5mm thus forming a cylindrical transmucosal element of 5.0 to 6.00 mm in diameter (fig. 4-6).

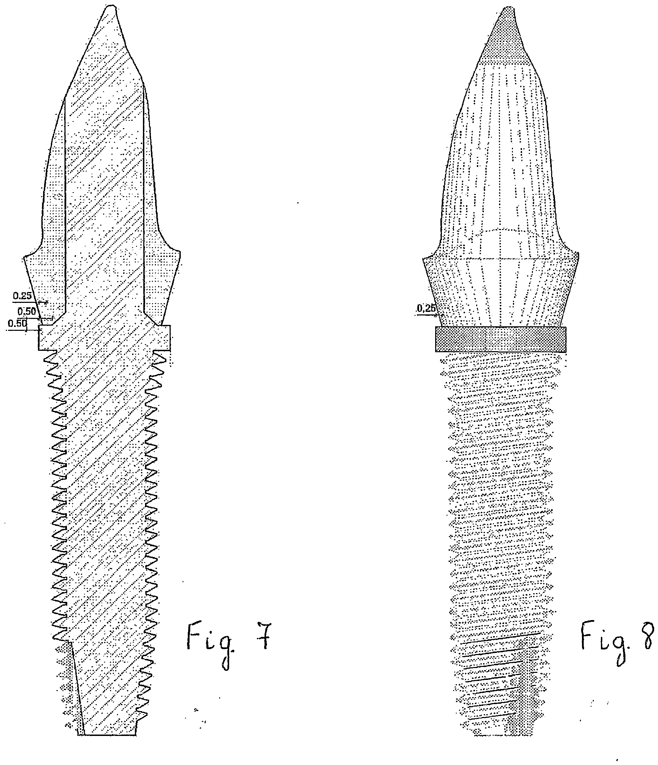

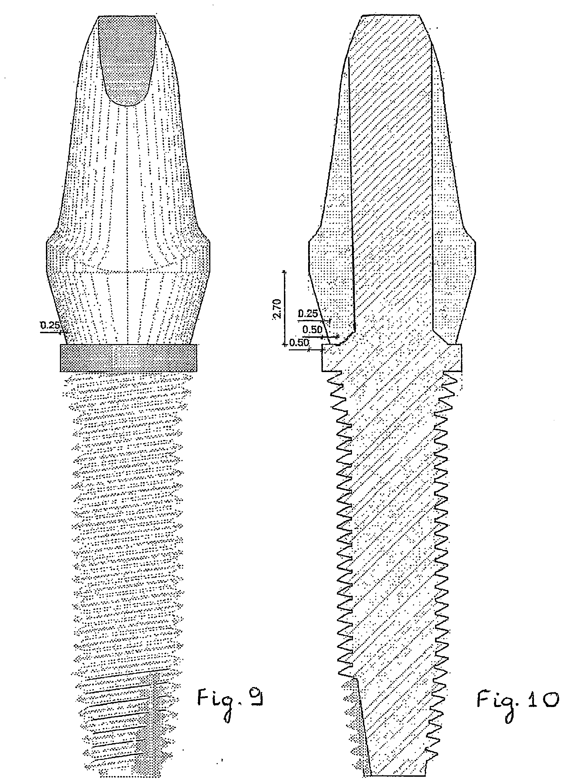

In the restorative abutment part the implant material that extends coronally and acts as the reinforcing core of the transmucosal and crown part of the restoration has a total height that varies from 10.0 mm to 15.0 mm. Coronally, its tip with rounded edges, is not covered by the coating and provides an external grip (1.5 mm in diameter hexagonal and 2mm in height) to be engaged by the rotating instrument that is used during the insertion by screwing the fixture into the prepared osseous bed. The coating material that covers the transmucosal part extends on the coronal part as well, in a uniform thickness of 1.0 to 1,5mm providing an amendable by tooth preparation shell in the restorative level of the abutment (fig. 7-10).

## Claims

1. One-piece dental implant consisting of a Ti or Zirconia endosseous screw shaped part which can be fitted in the jaw bone, bearing a transmucosal narrower extension core from the same material as the endosseous screw shaped part which extends transmucosally and coronally, forming a cylindrical or prismatic with a polygonal cross-sectional projection, acting as the supporting core of the intraoral abutment, **characterized in that** the transmucosal narrower extension core and its projection are coated by an esthetic ceramic or glass-ceramic material that is bonded on the entire transmucosal and intraoral core projection by fusion or heat pressing or by means of an adhesive.

## Patentansprüche

1. Einteiliges Zahnimplantat, das aus einem enossalen schraubenförmigen Abschnitt aus Titan oder Zirkoniumdioxid besteht, welcher in den Kieferknochen eingepasst werden kann, der ein transmukosal schmälere Ansatzstück, das sich transmukusal und koronal erstreckt, aus dem gleichen Material wie der enossale schraubenförmige Abschnitt trägt, welches ein zylinderförmiges oder ein prismatisches Verlängerungsteil mit einem polygonalen Querschnitt bildet, das als Stützkörper des intraoralen Pfeilers wirkt, **dadurch gekennzeichnet, dass** das transmukosal schmälere Ansatzstück und sein Verlängerungsteil mit einem ästhetischen Keramik- oder Glaskeramik-Material beschichtet sind, welches über das gesamte transmukosale und intraorale verlängerungsstück mittels Fusion oder Heißpressen oder mittels eines Klebstoffs verbunden ist.

## Revendications

1. Implant dentaire monobloc consistant en un composant façonné avec implant vis en zirconium ou titane qui peut être adapté à la mâchoire, supportant un noyau d' extension plus étroit transmucosal du même matériau que le composant façonné avec implant vis qui s'étend de manière transmucosale et coronaire, formant un cylindre ou prisme avec projection en coupe polygonale, agissant comme noyau de support du point d'appui intraoral, **caractérisé en ce que** le noyau d'extension plus étroit transmucosal et sa projection sont recouverts d'un matériau céramique esthétique ou en vitrocéramique qui est relié à la projection du noyau transmucosal ou intraoral entier par fusion ou pression à chaud ou au moyen d'un adhésif.
